# EUROPEAN PATENT SPECIFICATION

(11) **EP 0 652 227 B1**
(45) Date of publication and mention of the grant of the patent: **25.02.1998**
(21) Application number: 94914582.5
(22) Date of filing: 28.04.1994
(51) Int. Cl.: C07K 1/14, C07K 14/00

(54) **METHOD OF PURIFYING HYDROPHOBIC POLYPEPTIDE**
VERFAHREN ZUR REINIGUNG EINES HYDROPHOBEN POLYPEPTIDES
PROCEDE POUR PURIFIER UN POLYPEPTIDE HYDROPHOBE

(30) Priority: 30.04.1993 JP 103957/93
(43) Date of publication of application: 10.05.1995
(73) Proprietor: TOKYO TANABE COMPANY LIMITED, Chuo-ku Tokyo 103 (JP)
(72) Inventor: TAKEI, Tsunetomo, Tokyo Tanabe Company Limited, Kita-ku, Tokyo 115 (JP); AIBA, Toshimitsu, Tokyo Tanabe Company Limited, Kita-ku, Tokyo 115 (JP); SAKAI, Kaoru, Tokyo Tanabe Company Limited, Kita-ku, Tokyo 115 (JP); FUJIWARA, Tetsuro, Morioka-shi, Iwate 020 (JP)
(74) Representative: Skailes, Humphrey John
(86) International application number: PCT/JP94/00731
(87) International publication number: WO 94/25480

(56) References cited:
- WO-A-92/00993
- JP-A- 1 121 299
- JP-A- 5 294 996
- JP-T-63 501 792
- CHEMICAL ABSTRACTS, vol. 104, no. 25, 23 June 1986, Columbus, Ohio, US; abstract no. 218500a, G TAMAI ET AL. 'A new deproteinization method for high performance liquid chromatographic analysis of drugs with direct serum sample injection' page 4 ;
- JOURNAL OF CHROMATOGRAPHY, vol.476, 1989, AMSTERDAM NL pages 499 - 511 G THEVENON & F E REGNIER 'Reversed-phase liquid chromatography of proteins with strong acids'

### SEQUENCE LISTING

### (1) GENERAL INFORMATION:

(i) APPLICANT:
   (A) NAME: Tokyo Tanabe Company Limited
   (B) STREET: 2-6, Nihonbashi-honcho 2-chome,
   (C) CITY: Chuo-ku, Tokyo
   (D) STATE:
   (E) COUNTRY: Japan
   (F) POSTAL CODE (ZIP): 103
(ii) TITLE OF INVENTION: Purification method for hydrophobic polypeptides
(iii) NUMBER OF SEQUENCES: 11
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: WordPerfect Windows 6.0
(v) CURRENT APPLICATION DATA:
   APPLICATION NUMBER: EP 94914582.5
(vi) PRIOR APPLICATION DATA:
   (A) APPLICATION NUMBER: JP 103957/1993
   (B) FILING DATE: 30 April 1993

### (2) INFORMATION FOR SEQ ID NO: 1

(i) SEQUENCE CHARACTERISTICS:
   (A) LENGTH : 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 1
   Cys Pro Val His Leu Lys Arg Leu Leu Ile Val Val Val Val Val Val
   1               5                   10                  15
   Leu Ile Val Val Val Ile Val Gly Ala Leu Leu
               20                  25

### (3) INFORMATION FOR SEQ ID NO: 2

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
   (ii) MOLECULE TYPE: Peptide
   (xi) SEQUENCE DESCRIPTION: SEQ ID NO: 2
   Cys Cys Pro Val His Leu Lys Arg Leu Leu Ile Val Val Val Val Val
   1               5                   10                  15
   Val Leu Ile Val Val Val Ile Val Gly Ala Leu
               20                  25

### (4) INFORMATION FOR SEQ ID NO: 3

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 3
   Cys Pro Val Asn Ile Lys Arg Leu Leu Ile Val Val Val Val Val Val
   1               5                   10                  15
   Leu Leu Val Val Val Ile Val Gly Ala Leu Leu
               20                  25

### (5) INFORMATION FOR SEQ ID NO: 4

(i) SEQUENCE CHARACTERISTICS;
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 4
   Cys Cys Pro Val Asn Ile Lys Arg Leu Leu Ile Val Val Val Val Val
   1               5                   10                  15
   Val Leu Leu Val Val Val Ile Val Gly Ala Leu
               20                  25

### (6) INFORMATION FOR SEQ ID NO: 5

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(XI) SEQUENCE DESCRIPTION: SEQ ID NO: 5
   Cys Pro Val Asn Leu Lys Arg Leu Leu Val Val Val Val Val Val Val
   1               5                   10                  15
   Leu Val Val Val Val Ile Val Gly Ala Leu Leu
               20                  25

### (7) INFORMATION FOR SEQ ID NO: 6

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 6
   Cys Cys Pro Val Asn Leu Lys Arg Leu Leu Val Val Val Val Val Val
   1               5                   10                  15
   Val Leu Val Val Val Val Ile Val Gly Ala Leu
               20                  25

### (8) INFORMATION FOR SEQ ID NO: 7

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 7
   Ser Pro Val His Leu Lys Arg Leu Leu Ile Val Val Val Val Val Val
   1               5                   10                  15
   Leu Ile Val Val Val Ile Val Gly Ala Leu Leu
               20                  25

### (9) INFORMATION FOR SEQ ID NO: 8

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 8
   Ser Ser Pro Val His Leu Lys Arg Leu Leu Ile Val Val Val Val Val
   1               5                   10                  15
   Val Leu Ile Val Val Val Ile Val Gly Ala Leu
               20                  25

### (10) INFORMATION FOR SEQ ID NO: 9

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 9
   Ala Pro Val His Leu Lys Arg Leu Leu Ile Val Val Val Val Val Val
   1               5                   10                  15
   Leu Ile Val Val Val Ile Val Gly Ala Leu Leu
               20                  25

### (11) INFORMATION FOR SEQ ID NO: 10

(i) SEQUENCE CHARACTERISTICS:
   (A) LENGTH: 27 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 10
   Ala Ala Pro Val His Leu Lys Arg Leu Leu Ile Val Val Val Val Val
   1               5                   10                  15
   Val Leu Ile Val Val Val Ile Val Gly Ala Leu
               20                  25

### (12) INFORMATION FOR SEQ ID NO: 11

(i) SEQUENCE CHARACTERISTICS:
   (A) LENGTH: 35 Amino acids
   (B) TYPE: Amino acid
   (C) STRANDEDNESS: Single
   (D) TOPOLOGY: Linear
(ii) MOLECULE TYPE: Peptide
(xi) SEQUENCE DESCRIPTION: SEQ ID NO: 11

## Description

### Technical Field of the Invention

The present invention relates to a purification method for hydrophobic polypeptides, and more particularly to a purification method for hydrophobic polypeptides which show a more intensive surface activity in combination with a lipid mixture.

### Background Art

Infantile respiratory distress syndrome is a disease which induces serious respiratory disorder as a result of the collapse of lung due to a deficiency of pulmonary surfactant and is observed in premature newborns with high mortality. In recent years, for the treatment of this respiratory distress syndrome, a supplemental therapy in which pulmonary surfactant is administered via a transairway route from the outside has been developped, and this therapy has demonstrated successful results.

Some of the inventors of the present invention isolated lipoprotein from pulmonary surfactant derived from animals, and they have found that the lipoprotein is an essential component for showing pulmonary surface activity, and that pulmonary surfactant including the complex lipid-lipoprotein mixture prepared by combining said lipoprotein with a lipid mixture showed excellent surface activity, namely the surface tension reducing effect, and to provide an alveolar space volume sufficient to maintain normal respiratory function by shortening the niveau surface spreading time and low equilibrium surface tension, thereby making the lipoprotein suitable for the treatment of respiratory distress syndrome (See Japanese Patent Publication No. Hei 3-78371 Gazette).

Furthermore, the inventors of the present invention found that a synthesized polypeptide having a partial structure of apoprotein C (sequence number 11, hereinafter referred to as "SP-C"), which is a specific apoprotein of the pulmonary surface of mammals, shows strong surface activity when it is combined with a lipid mixture (Japanese Patent Application No. Hei 4-98083).

However, purification of SP-C and the synthesized polypeptide described above having partial structure of SP-C was very difficult due to the extremely low solubility of these polypeptides in the moving phase, which is due to their very high hydrophobic properties under the conditions of normal high-performance liquid chromatography (hereinafter referred to as "HPLC").

In general, a hydrophobic protein or polypeptide is defined as one having a relatively high content of hydrophobic amino acids (including isoleucine, tyrosine, phenylalanine, leucine, valine and methionine) in the component amino acid residues. In Table 1, the total number of amino acid residues in certain proteins, the number of hydrophobic amino acid residues and hydrophobic amino acid content (number of hydrophobic amino acid residues/total number of amino acid residues) are shown. As can be seen from the table, both surfactant apoprotein B (hereinafter referred to as "SP-B") and SP-C are highly-hydrophobic proteins as compared to the other proteins.

**TABLE 1**

| **Protein** | **Total number of Amino Acids** | **Number of Hydrophobic Amino Acids** | **Content of Hydrophobic Amino Acids (%)** |
|---|---|---|---|
| Glutamic dehydrogenase (Bovine, liver) | 500 | 199 | 39.8 |
| Aldolase A-Chain (Rabbit, myocardium) | 361 | 148 | 41.0 |
| Lysozyme (Chicken, albumin) | 129 | 45 | 34.9 |
| Trypsine (Bovine, spleen) | 229 | 77 | 33.6 |
| Pepsin (Porcine) | 327 | 128 | 39.1 |
| Basic Trypsinse Inhibitor (Bovine, spleen) | 58 | 20 | 34.5 |
| Insulin A, B-Chain (Human) | 41 | 17 | 41.5 |
| Glucagon (Human) | 29 | 8 | 27.6 |
| Calcitonin (Human) | 32 | 12 | 37.5 |
| HDL-apoA-I (Human) | 245 | 94 | 38.4 |
| HDL-apoA-II (Human) | 77 | 29 | 37.7 |
| Myosin A1 L-Chain (Rabbit, skeletal muscle) | 190 | 84 | 44.2 |
| κ-casein B Variant (Bovine, milk) | 169 | 74 | 43.8 |
| Serum Albumin (Human) | 585 | 234 | 40.0 |
| Basic Protein (Human, myelin membrane) | 171 | 53 | 31.0 |
| SP-B | 57 | 36 | 63.2 |
| SP-C | 35 | 27 | 77.1 |

For purification of such hydrophobic polypeptides, a method using reversed-phase HPLC which uses a polar solvent, such as H₂O, acetonitrile and methanol, as the moving phase, has been widely adopted.

For the moving phase, reversed-phase HPLC using, for example, a mixed solvent obtained by adding an organic acid (such as formic acid or trifluoroacetic acid) to a polar solvent (such as H₂O), acetonitrile or methanol) is known, but only a trace amount of trifluoroacetic acid as small as 0.5% is used (Japanese Patent Laid-open No. Hei 1-501282 Gazette).

On the other hand, it was difficult to carry out reversed-phase HPLC in the purification of hydrophobic proteins such as SP-B and SP-C, which are contained in pulmonary surface active agent derived from animals, using this solvent as the moving phase because of the low solubility of the proteins in the moving phase.

Moreover, it was not possible to avoid the contamination of the hydrophobic polypeptide with a trace amount of impurity, even with polyvinyl alcohol-based HPLC column filler and using the aforementioned polar solvents as the moving phase. When such a contaminant is present, the pulmonary surfactant is colored, and the hydrophobic polypeptide cannot demonstrate sufficient surface activity as it polymerizes and coagulates.

In view of the above, the development of an improved purification method has been required.

### Disclosure of the Invention

The inventors of the present invention have carried out intensive investigations to find a method for purifying hydrophobic polypeptides, and they have found an excellent method wherein the hydrophobic polypeptide is dissolved in trifluoroacetic acid (hereinafter referred to as "TFA") and is applied to HPLC in which a mixed solvent containing TFA is used as the moving phase and polyvinyl alcohol-based column filler is used.

For the mixed solvent to be used as the moving phase, a mixed solvent containing TFA at a concentration of 3 to 10%, preferably 5 to 10%, and most preferably 7 to 10%, may be used. For the solvent to be used as the mixed solvent, halogenated hydrocarbons, such as dichloromethane, chloroform and 1,2-dichloroethane, are preferably used. If amino acids having a thiol group, such as cysteine, are present in the peptide, β-mercaptoethanol or the like may be added as an antioxidant for the thiol group. The quantity of β-mercaptoethanol to be added is generally such as to provide a final concentration of 0.1 mM.

The purification method of the present invention, can also be used to purify a wide range of other hydrophobic polypeptides as well as the other hydrophobic lipoprotein (eg. SP-B) contained in pulmonary surfactant.

### BEST MODE FOR CARRYING OUT THE INVENTION

The present invention will be further explained with reference to the examples.

In the examples, 0.05 to 5 mg of crude polypeptide with sequence number 1 synthesized according to solid-phase method was subjected to HPLC for 75 minutes using an Asahipak GS-510 (diameter 7.5x500mm) column (trademark, Asahi Kasei Co. Ltd.) and a flow rate of 0.8ml/min.

The result of the purification was evaluated from the color of the polypeptide obtained and the surface activity properties of the pulmonary surfactant prepared from said polypeptide.

For determining the color of the polypeptide, a TFA solution was prepared.

The preparation of pulmonary surfactant was carried out according to the following procedure. To each of the samples of purified polypeptide in solution in TFA (12mg/0.5ml) was added 100ml of chloroform-methanol solution (2:1, v/v) containing 1,2-dipalmitoylglycero-(3)-phosphocholine (660mg), 1,2-diacyl-sn-glycero-(3)-phospho-sn-glycerol (220mg) and palmitic acid (100mg) and the mixture obtained was then dried and solidified under reduced pressure. The residue obtained was suspended in 100 ml of H₂O-ethanol mixture (9:1, v/v) at a temperature of 40°C for 15 minutes,and then lyophilized for 36 hours at -50°C and a vacuum of 85 to 100 µHg to prepare a pulmonary surfactant.

The surface activity was evaluated from the measurement of the surface tension reducing effect, spreadability over a gas-liquid interface and adsorbability at a gas-liquid interface.

### Reference Example 1

10 mg of polypeptide of sequence number 1 (hydrophobic amino acid content: 81.4%) was dissolved in formic acid, and methanol-H₂O solution (70:30, v/v) containing 10mM β-mercaptoethanol (which is the solvent to be used as the moving phase) was then added thereto to prepare sample solution at a concentration of 2 mg/ml.

100 µl of the sample solution was injected into a column equilibrated with the aforementioned moving phase and subjected to HPLC in said moving phase. The detection was carried out at 250 nm, and the sample solution was divided into 15 fractions. Each fraction was then dried and solidified under reduced pressure, and the dry weight thereof was measured. From each purified polypeptide, a pulmonary surface was prepared, and its surface activity was determined.

The polypeptide obtained appears yellowish when dissolved in TFA. The yield of active fraction was 37%.

Surface tension reducing effect:
Maximum surface tension 33.8 dyne/cm
Minimum surface tension 4.1 dyne/cm

Spreadability over a gas-liquid interface:
Equilibration time 60 seconds
Equilibrated surface tension 4.1 dyne/cm

Adsorbability to a gas-liquid interface:
Equilibration time 90 seconds
Equilibrated surface tension 32.6 dyne/cm

(The results are those of the fractions which showed the best activity. The same was applied to the following.)

### Example 1

20 mg of polypeptide of sequence number 1 was dissolved in 1 ml of TFA. The resulting solution was processed according to the procedure as described in the Reference Example 1 except TFA-dichloromethane solution (10:90, v/v) containing 10 mM of "β-mercaptoethanol was used as the moving phase.

The polypeptide obtained has no color when dissolved in TFA and the yield of the active fraction was 66%.

The surface activity properties of the pulmonary surfactant containing this polypeptide are as follows.

Surface tension reducing effect:
Maximum surface tension 29.5 dyne/cm
Minimum surface tension 2.7 dyne/cm

Spreadability over a gas-liquid interface:
Equilibration time 30 seconds
Equilibrated surface tension 29.4 dyne/cm

Adsorbability to a gas-liquid interface:
Equilibration time 40 seconds
Equilibrated surface tension 30.1 dyne/cm

### Example 2

20 mg of polypeptide (hydrophobic amino acid content: 81.4%) of sequence number 1 was dissolved in 1 ml of TFA. The resulting solution was processed according to the procedure as described in the Example 1 except TFA-dichloromethane solution (3:97, v/v) was used as the moving phase.

The polypeptide obtained has no color when dissolved in TFA and the yield of the active fraction was 42%.

The surface activity properties of the pulmonary surfactant containing this polypeptide is as follows.

Surface tension reducing effect:
Maximum surface tension 28.9 dyne/cm
Minimum surface tension 2.1 dyne/cm

Spreadability over a gas-liquid interface:
Equilibration time 40 seconds
Equilibrated surface tension 29.0 dyne/cm

Adsorbability to a gas-liquid interface:
Equilibrated time 50 seconds
Equilibrated surface tension 31.4 dyne/cm

### Example 3

The polypeptide of sequence number of 11 was purified according to the procedure as described in Example 2. It was observed that TFA solutions of the polypeptide obtained had no color.

## Claims

1. A purification method for hydrophobic polypeptides using high performance liquid chromatography characterized by using halogenated hydrocarbon containing from 3 to 10% by volume of trifluoroacetic acid as the moving phase and a polyvinyl alcohol-based column filler, and in that the hydrophobic polypeptide is one whose hydrophobic amino acid content is more than 50%.

2. A method according to claim 1 wherein the moving phase also contains β-mercaptoethanol.

3. A method according to claim 1 or claim 2 wherein the hydrophobic polypeptide is a pulmonary surfactant peptide.

4. A method according to any of claims 1 to 3 wherein the hydrophobic polypeptide is as defined by any one of the sequence numbers 1 to 11 below:

## Patentansprüche

1. Reinigungsverfahren für hydrophobe Polypeptide unter Verwendung von Hochdruck-Flüssigkeitschromatographie, **gekennzeichnet** durch die Verwendung eines halogenierten Kohlenwasserstoffes, enthaltend von 3 bis 10 Vol-% Trifluoressigsäure als bewegliche Phase und einen Säulenfüllstoff auf Polyvinylalkohol-Basis, und dadurch **gekennzeichnet**, daß das hydrophobe Polypeptid eines ist, dessen hydrophober Aminsäuregehalt höher ist als 50%.

2. Verfahren nach Anspruch 1, worin die bewegliche Phase ebenfalls β-Merkaptoethanol enthält.

3. Verfahren nach Anspruch 1 oder 2, worin das hydrophobe Polypeptid ein oberflächenaktives Peptid ist.

4. Verfahren nach einem der Ansprüche 1 bis 3, worin das hydrophobe Polypeptid definiert ist nach einem der Sequenznummern 1 bis 11 wie unten angegeben:

## Revendications

1. Procédé de purification de polypeptides hydrophobes utilisant la chromatographie liquide à haute performance, caractérisé par l'utilisation d'hydrocarbure halogéné contenant de 3 à 10 % en volume d'acide trifluoroacétique comme phase mobile et une charge de colonne à base d'alcool polyvinylique, et en ce que le polypeptide hydrophobe a une teneur en acides aminés hydrophobes supérieure à 50 %.

2. Procédé selon la revendication 1 dans lequel la phase mobile contient en outre du β-mercaptoéthanol.

3. Procédé selon la revendication 1 ou la revendication 2, dans lequel le polypeptide hydrophobe est un peptide du surfactant pulmonaire.

4. Procédé selon l'une quelconque des revendications 1 à 3, dans lequel le polypeptide hydrophobe est tel que défini par l'une quelconque des séquences numérotées de 1 à 11 ci-dessous :
